# EUROPEAN PATENT APPLICATION

(11) **EP 2 878 287 A1**
(43) Date of publication of application: **03.06.2015**
(21) Application number: 13195297.0
(22) Date of filing: 02.12.2013
(51) Int. Cl.: A61F 7/10, A61F 7/00, A61F 7/02

(54) **Device for cooling scalp**

(71) Applicant: Arline Beheer B.V., 3972 XN Driebergen (NL)
(72) Inventor: Arline Beheer B.V., 3972 XN Driebergen (NL)
(74) Representative: Luten, Martin Haaije

(57) **Abstract**

The invention relates to a device for cooling scalp with a cooling fluid, which device comprises:
- a skull cap having an inner wall, an outer wall and a plurality of connecting walls for connecting the inner wall with the outer wall while defining cooling channels there between,
- at least one inlet connector connected to the cooling channels for supplying a cooling fluid to the cooling channels;
- at least one outlet connector connected to the cooling channels for discharging the cooling fluid from the cooling channels;
wherein the channels in the skull cap extend from the forehead edge, over the top zone of the cap to the neck edge of the skull cap.

## Description

The invention relates to a device for cooling scalp with a cooling fluid, which device comprises:
- a skull cap having an inner wall, an outer wall and a plurality of connecting walls for connecting the inner wall with the outer wall while defining cooling channels there between,
- at least one inlet connector connected to the cooling channels for supplying a cooling fluid to the cooling channels; and
- at least one outlet connector connected to the cooling channels for discharging the cooling fluid from the cooling channels.

Such a device is for example known from NL 9000752 and is used to cool the scalp of a person. The cooling of the scalp has as result, that the blood circulation is reduced.

In case of chemotherapy, the reduction in blood circulation in the scalp at the moment the cytostatics is injected into a person has the advantage that the hair loss, which is a typical side effect of chemotherapy, is reduced considerable.

In order to achieve a controlled cooling of the scalp, it is necessary that the skull cap has a tight fit. This will ensure that the scalp is cooled uniformly. If no uniform cooling is achieved, this would result in an uneven reduction of blood circulation, causing discomfort to the person wearing the skull cap. In case of chemotherapy, it would also result in an uneven hair loss.

Another problem is that the size of a persons skull varies requiring different sizes of skull caps to achieve a tight fit of the skull cap on the head of a specific person.

NL 9000752 teaches to manufacture the skull cap out of a deformable material, but states that to have a better fit to the different sizes of heads, an inflatable cushion is provided between a rigid outer shell and the cooling element, such that the cushion can be pushed against the head.

Besides a complex construction of the device, the cooling element also has to be larger than the head to be able to be pushed by the cushion against the head of the wearer.

Furthermore, the cooling element of this prior art device has zones, in which the channels are directed in different directions. Each zone of channels has different elasticity in the main directions of the zone. As the channels of the zones are directed in different directions, this will impede the flexibility of the full skull cap, in addition to reduced flexibility of the rigid outer shell.

US 4566455 describes a cooling cap having a tube running in circles around the head. After the tube is arranged around the head, a flexible shower cap like part is pulled over the tube.

Although the shower cap is flexible, the diameter of the circle shaped tube defines the fit of the cooling cap. If a too small cooling cap would be arranged on a head, then the tube has to be stretched in longitudinal direction, which is typically the most rigid main direction of a tube.

The object of the invention is to reduce the above mentioned disadvantages.

This object is achieved according to the invention with a device according to the preamble, which device is characterized in that the channels in the skull cap extend from the forehead edge, over the top zone of the cap to the neck edge of the skull cap.

A skull cap typically has an edge running along the forehead of the wearer, then along one ear side to the neck, where the edge will return along the other ear of the wearer back to the forehead. From this edge, the skull cap will have a dome shape, wherein the upper part of the dome shape can be called a top zone.

With the device according to the invention, the channels extend from the forehead edge, over the top zone of the cap to the neck edge of the skull cap. When the skull cap is worn, the channels will run substantially perpendicular to the plane extending through the forehead and neck of the wearer, which will typically be the largest diameter of the head. If the skull cap needs to be radially expanded to fit around this largest diameter, the channels will be subjected to a stretch force perpendicular to the length of the channel. If the channel would have a circular cross section, then the cross section would become oval as a result of this stretch force. The length of the channel would not chance substantially. So, the required expansion length is obtained from reducing the height of the channels, making the skull cap more flexible than the prior art skull caps, in which the channels need to be stretched in length direction.

Preferably, the skull cap is of an elastic material, such as silicone. Silicone material is very flexible and can be easily managed during manufacturing of the skull cap.

In a preferred embodiment of the device according to the invention the cooling channels are parallel to each other. By arranging the cooling channels parallel, an even distribution of the cooling fluid is achieved and furthermore, the strechability of the cooling cap is more uniform.

In another embodiment of the device according to the invention the inner wall, outer wall and the plurality of connecting walls are formed by flexible hoses.

Flexible hoses are readily available and one can easily manufacture a device according to the invention at low costs. Furthermore, by using a hose, it is ensured that no leakage will occur over the length of the cooling channels.

In a very preferred embodiment of the device according to the invention adjacent channels are alternately connected to each other near the forehead edge and the neck edge to form meandering channels.

With the meandering channels the channel will have a return part, such that supply and discharge of a cooling channel can be at the same location, for example in the neck of the wearer, which will increase the wear comfort.

In still further preferred embodiment of the device according to the invention the meandering channels are divided into separate zones and wherein additional inlet connectors are provided for separately supplying cooling fluid to the meandering channel zones.

Having different zones of meandering cooling channels provides the possibility to cool parts of the scalp further than other parts or to provide more cooling to parts of the scalp, which produce more heat.

Preferably, the separate meandering channel zones are connected to the at least one outlet connector. As soon as the cooling fluid has flowed through the different cooling channel zones it can be collected at one outlet connector, where the fluid is recirculated to a cooling device, to be cooled again and be supplied again to the channels.

Yet another embodiment of the device according to the invention comprises at least one temperature sensor extending from the inner wall into the inner space of the skull cap.

The at least one temperature sensor can be used to measure the temperature of the scalp to regulate the cooling of the device.

Preferably, the temperature sensitive part of the temperature sensor is suspended flexibly. The flexible suspension of the temperature sensitive part enables a good contact with the scalp without being pushed too hard into the scalp, which could cause discomfort to the wearer.

These and other features of the invention will be elucidated in conjunction with the accompanying drawings.
Figure 1 shows a side view of a device according to the invention.
Figure 2 shows a back view of the device of figure 1.
Figure 3 shows the inner wall of the device according to figure 1.
Figure 4 shows the outer wall with the connecting walls of figure 1.

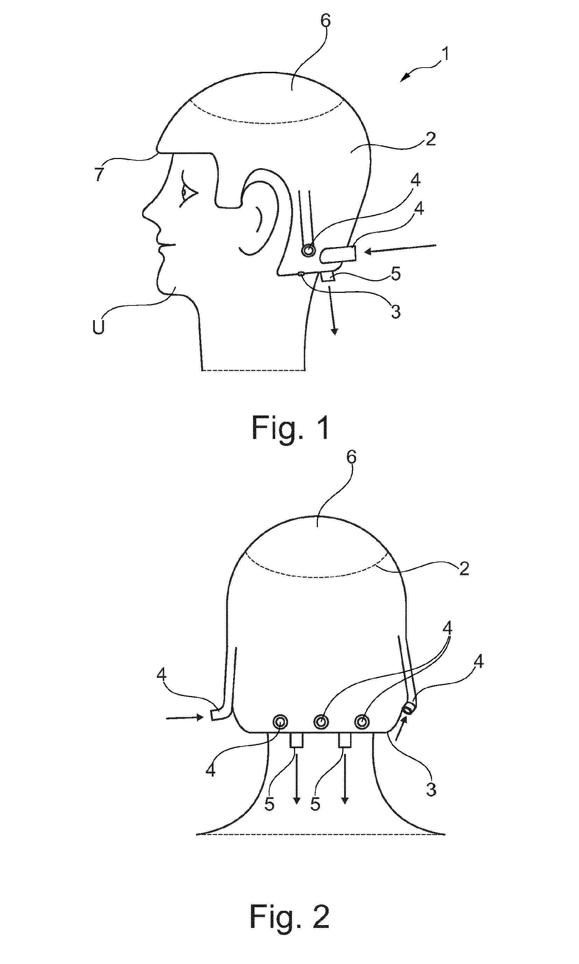

Figure 1 shows a side view of a device 1 according to the invention. The device 1 is worn by a user U. The device 1 has a skull cap 2, with near the neck edge 3 a five inlet connectors 4 and two outlet connectors 5. (See also figure 2) The skull cap 2 had a top zone 6 and a forehead edge 7.

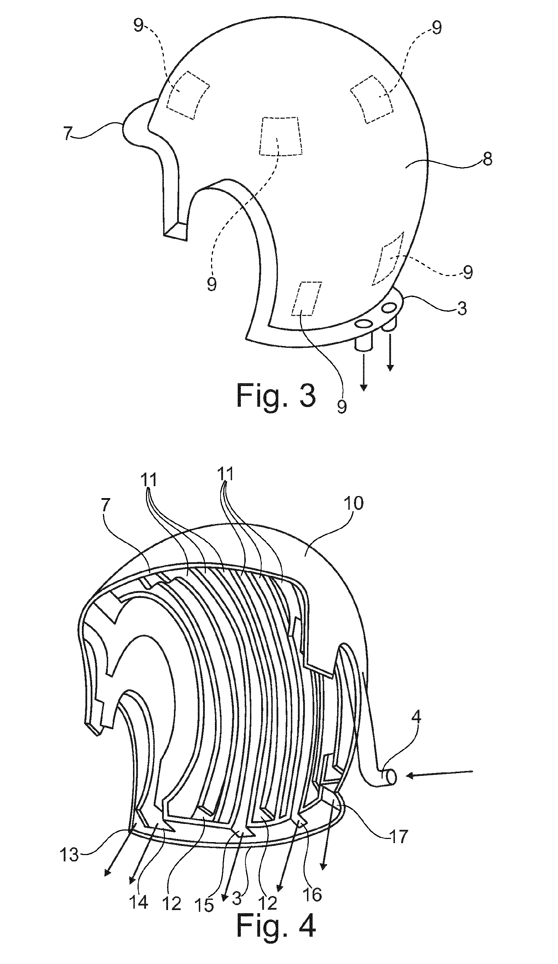

Figure 3 shows the inner wall 8 of the device 1 according to figure 1. The device 1 is preferably build out of two injection molded parts 8, 10 made of a flexible material, such as silicone.

The inner wall 8 has on the side directed to the scalp of the wearer U a number of temperature sensors 9 for registering the temperature of the scalp. Based on these measurements more of less cooling fluid can be fed to the inlet connectors 4. For example a controller, like a computer program, can be used, in which the desired temperature for each zone around each temperature sensor 9 can be set. The controller will then supply more of less cooling fluid to each separate cooling zone (further explained below) to achieve the desired temperature. It would also be possible to regulate the temperature of the cooling fluid supplied to each zone, in order to control the desired temperature of each zone. The controller uses the set temperature and the measured temperature in a suitable control, like a PID control.

Figure 4 shows the outer wall 10 of the device of figure 1. A plurality of connecting walls are arranged on the inside of the outer wall 10, such that a plurality of parallel channels 11 are provided. These channels 11 extend from the forehead edge 7, over the top zone 6 of the cap 2 to the neck edge 3 of the skull cap 2. The channels 11 are connected near the neck edge 3 or the forehead edge 7 with intermediate channels 12 to provide meandering channels. This ensures that separate meandering channels are provided, which are fed with a cooling fluid via the inlet connectors 4 and end at the openings 13, 14, 15, 16, 17 after which the fluid is collected in a space near the neck edge 3.

The inner wall 8 is a preferably a separate injection molded piece and the outer wall 10 with the connecting walls is another separate injection molded piece. Both separate pieces 8, 10 are joined together, for example by welding or by adhesion, to form a liquid tight skull cap 2 having an increased flexibility, such that it will tightly fit on a larger number of sizes of heads.

## Claims

1. Device for cooling scalp with a cooling fluid, which device comprises:
- a skull cap having an inner wall, an outer wall and a plurality of connecting walls for connecting the inner wall with the outer wall while defining cooling channels there between,
- at least one inlet connector connected to the cooling channels for supplying a cooling fluid to the cooling channels;
- at least one outlet connector connected to the cooling channels for discharging the cooling fluid from the cooling channels;
**characterized in that**
the channels in the skull cap extend from the forehead edge, over the top zone of the cap to the neck edge of the skull cap.

2. Device according to claim 1, wherein the skull cap is of an elastic material, such as silicone.

3. Device according to claim 1 or 2, wherein the cooling channels are parallel to each other.

4. Device according to claim 3, wherein the inner wall, outer wall and the plurality of connecting walls are formed by flexible hoses.

5. Device according to any of the preceding claims, wherein adjacent channels are alternately connected to each other near the forehead edge and the neck edge to form meandering channels.

6. Device according to claim 5, wherein the meandering channels are divided into separate zones and wherein additional inlet connectors are provided for separately supplying cooling fluid to the meandering channel zones.

7. Device according to claim 6 wherein the separate meandering channel zones are connected to the at least one outlet connector.

8. Device according to any of the preceding claims, further comprising at least one temperature sensor extending from the inner wall into the inner space of the skull cap.

9. Device according to claim 8, wherein the temperature sensitive part of the temperature sensor is suspended flexibly.
